# EUROPEAN PATENT SPECIFICATION

(11) **EP 0 662 145 B1**
(45) Date of publication and mention of the grant of the patent: **02.01.1997**
(21) Application number: 93917597.2
(22) Date of filing: 20.07.1993
(51) Int. Cl.: C12P 21/04

(54) **PROCESS FOR THE SELECTIVE INCREASE OF PRODUCTION OF ANTIBIOTIC GE 2270 FACTOR A**
VERFAHREN ZUR SELEKTIVEN VERBESSERTEN HERSTELLUNG DES ANTIBIOTIKUM GE 2270 FAKTOR A
PROCEDE UTILE POUR AUGMENTER SELECTIVEMENT LA PRODUCTION DU FACTEUR A DE L'ANTIBIOTIQUE GE 2270

(30) Priority: 10.09.1992 EP 92115451
(43) Date of publication of application: 12.07.1995
(73) Proprietor: GRUPPO LEPETIT S.P.A., 20020 Lainate (MI) (IT)
(72) Inventor: RIZZO, Angelo, Michele, I-72023 Mesagne (IT); GASTALDO, Luciano, I-20010 Pogliano Milanese (IT)
(74) Representative: Macchetta, Francesco
(86) International application number: EP9301907
(87) International publication number: WO9405798

(56) References cited:
- EP-A- 0 359 062
- JOURNAL OF ANTIBIOTICS vol. 44, no. 7, July 1991, TOKYO JP pages 693 - 701 E. SELVA ET AL 'Antibiotic GE2270 A : a novel inhibitor of bacterial protein synthesis' cited in the application s
- Section Ch, Week 8003, 5 December 1979 Derwent Publications Ltd., London, GB;
- Class B02, AN 80-04482C &
- Journal of Antibiotics, vol.47, no. 12, pp 1564-1567 (1994)

## Description

Antibiotic GE 2270 is a thiazolyl peptide substance isolated from a culture of Planobispora rosea ATCC 53773.

This substance, which is mainly active against gram positive bacteria as well as some gram negative anaerobes, is described in European Patent Application Publication No. 359062 together with the process for obtaining it and the corresponding pharmaceutical compositions.

Antibiotic GE 2270 is produced by the microorganism as a complex of the closely related components A, B1, B2, C1, C2, C2a, D1, D2, E, and T which have been isolated and characterized.

Factor A, which is the component obtained in preponderant amount and the most relevant for the biological activity, is described in the above mentioned European Patent Application Publication No. 359062 together with the process for its isolation and its uses (see also: E. Selva et al., "Antibiotic GE 2270 A: A Novel Inhibitor of Bacterial Protein Synthesis. I. Isolation and Characterization". The Journal of Antibiotics, Vol. 44 No. 7, 693-701, 1991; J. Kettenring et al, "Antibiotic GE 2270 A: A Novel Inhibitor of Bacterial Protein Synthesis. II. Structure Elucidation". The Journal of Antibiotics, Vol.44 No.7, 702-715,1991).

Factor A is utilized as starting material for the preparation of antibiotic GE 2270 factors A1, A2, A3, (by hydrolysis) and H (by treatment with a reducing agent, e.g. NaBH₄).

Factors B1, B2, C1, C2, D1, D2, E, and T as well as the process for their isolation and uses are described in European Patent Application Publication No. 451486. Factor C2a and its process of isolation is described in European Patent Application Publication No. 529410 (corresponding to US Patent application Serial No. 07/931084).

The preparation of Antibiotic GE 2270 factors A1, A2, A3, and H from GE 2270 factor A is described in European Patent Application Publication No. 406745. See also E. Selva et al., " Natural Antibiotics Related to GE 2270 A.: Isolation, Structure Elucidation and Biological Characterization", 31st ICAAC, Sept. 29 - Oct. 2, 1991, Chicago. In said paper factor C2 is identified as C2b.

The structures of the components of the complex GE 2270 are set forth in Table I.

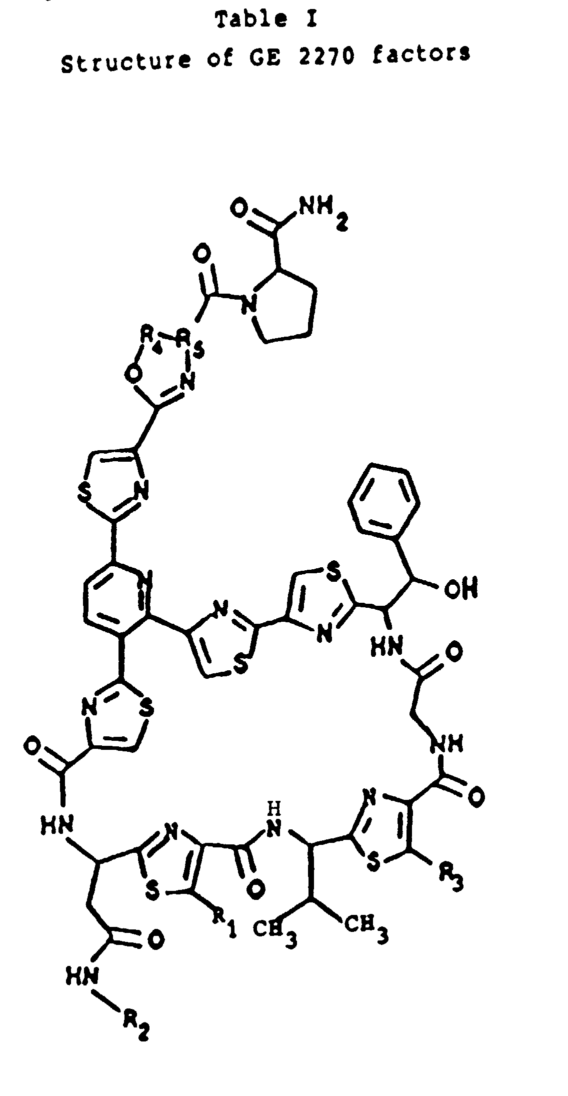

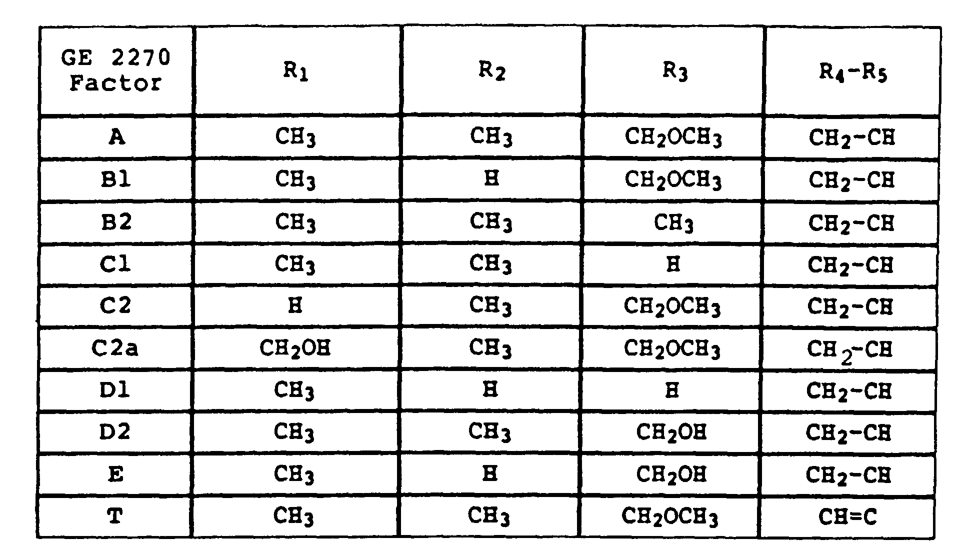

Due to the increasing development of tolerance and even resistance to current antibiotic treatments, the need for new antibiotic substances is still high. Particularly desirable are antibiotics in single isolated form with well defined and standardized composition assuming constant physico-chemical (e.g. stability) and biological (e.g. pharmacokinetics) behavior.

The current fermentation method described in EP-A 359062 yields a complex mixture wherein the factor A is generally less than 70% (by HPLC) with a total antibiotic productivity generally lower than 300 ppm. According to this invention it has been found that addition of vitamin B₁₂ (cyanocobalamin) or its analog having vitamin B₁₂ - like activity wherein the cyano group is replaced by another ligand, to the fermentation medium employed for production of the GE 2270 antibiotic complex enhances both the total antibiotic complex yield and the selectivity in the production of factor A.

Examples of analogs of vitamin B₁₂ having vitamin B₁₂ - like activity are, for instance, hydroxocobalamin, 5'-deoxyadenosyl-cobalamin, methylcobalamin as well as those listed in the book "THE VITAMINS. Chemistry, Physiology, Pathology, Methods" edited by W.H. Sebrell and R.S. Harris, Vol. II, pag. 181, Academic Press, New York - London, 1968.

Antibiotic production improvements due to the effect of vitamin B₁₂ are reported in literature for monensins, fortimicin, gentamycin, chuangxinmycin, SF-2312, antibiotic having heptenic acid skeleton (thienamycin, PS-5, etc), elsamicin and leinamycin.

However, it does not appear that a simultaneous double effect showing an increase of total antibiotic yield and a highly selective production of one of the components of the complex has been observed.

Detailed studies have been done on gentamycin producing strains of Micromonospora purpurea, by Russian authors (T.P. Krasnova et al.: "Comparative Studies on Active Strain of Micromonospora purpurea and its Low Active Mutant in Connection with Biosynthesis of Gentamycin". Antibiotics 24, No. 5, 323-328, 1979; Ringdoc Abstract 31044U). Their results could be summarized as follows:
(a) Additions of cobalt to the fermentation medium stimulates the biosynthesis of vitamin B₁₂ and influences the antibiotic complex composition.
(b) The mechanism proposed for the effect of the cobalt addition is that the cobalt ion stimulates the synthesis of the endogeneus vitamin B₁₂ necessary for the synthesis of methionine which is a source of methyl groups.

In experiments made by the inventors with GE 2270, only a moderate effect of CoCl₂ addition has been noted while the factor A selectivity was slightly influenced and the overall GE 2270 synthesis was slightly increased to values of the order of 300 ppm.

The amounts of vitamin B₁₂ or its analogs to be added to the fermentation medium in order to reach the desired effect according to this invention usually vary depending on the producing strain and composition of the culture medium utilized. Simple laboratory scale tests allow the person skilled in the art to determine the appropriate amounts of vitamin B₁₂ or its analogs to be added to the fermentation medium.

Under the usual fermentation conditions the effective amount generally ranges between 0.0005 and 5 ppm. Amounts larger than 5 ppm may be employed but without any substantial enhancement of the effect which can be reached with the upper value of the above interval. A preferred addition of vitamin B₁₂ ranges between 0.005 and 1 ppm.

The vitamin B₁₂ can be added as a free compound (e.g. crystalline vitamin B₁₂), as a concentrate or as a component of a more complex material which can be added to the fermentation medium. Said material should be acceptable for the fermentation conditions required for the production of the antibiotic GE 2270, i.e., it should not be toxic to the mentioned strain and should not negatively affect its growth and productivity.

Preferably, this material can be utilized by the microorganism also as an effective source of the essential elements for its growth, that is, it can be utilized also as source of carbon, nitrogen and/or mineral salts. For instance, vitamin B₁₂ activity is contained in both standard and de-fatted fish meal. Addition of an appropriate amount of fish meal to the fermentation medium can therefore act as an assimilable nitrogen source and simultaneously provide the necessary amount of vitamin B₁₂ to obtain the effect of this invention.

The fermentation media and the culturing process conditions utilized in the method of this invention are essentially those already described in the above mentioned EP-A 359062. Planobispora rosea ATCC 53773 or any antibiotic GE 2270 producing mutant or variant thereof is employed as antibiotic GE 2270 producing microorganism. Said variants or mutants may be obtained for instance by treatment with various known mutagens as indicated in the above mentioned EP-A 359062.

In summary, this invention consists in a fermentation process for the production of antibiotic GE 2270 complex in high yield and selectively enriched in the predominant factor A by cultivation of the strain Planobispora rosea ATCC 53773 or any antibiotic GE 2270 producing mutant or variant thereof under submerged aerobic conditions in the presence of assimilable sources of carbon, nitrogen and inorganic salts characterized in that vitamin B₁₂, or its analogs having vitamin B₁₂ - like activity wherein the cyano group is replaced by another ligand, is added to the fermentation medium.

As explained above, the fermentation procedures utilized according to this invention are essentially the same as those described in the state of the art for producing antibiotic GE 2270 complex. Frozen culture samples of Planobispora rosea ATCC 53773 are used to inoculate a flask or a vessel containing vegetative medium. The cultures are then incubated with shaking at a temperature between 26°C and 37°C for a sufficient time to achieve a desired growth. At a temperature between 28°C and 30°C an incubation time of 60-75 hours is generally sufficient for a satisfactory growth. After this period, aliquots of the obtained cultures are transferred into flasks or vessels containing the fermentation medium supplemented with the appropriate amounts of vitamin B₁₂ or its analogs. If the vitamin B₁₂ is added as a component of a complex material or, preferably, as a component of a nutrient material of the fermentation medium, said material should be dosed in relation to its content of vitamin B₁₂. The dosage required to obtain the effect of this invention may be determined by carrying out a series of cultivation experiments wherein different concentrations of the selected material are added to the fermentation medium or by pre-determining the contents of vitamin B₁₂ of the said material by solvent (i.e. benzyl alcohol, phenol or butanol) extraction and chromatographic separation/analysis of the extract.

However, in some cases, there is a limitation of the amount of material that can be added to the fermentation broth since above certain concentration values some materials show a general depressing effect on the microorganism producing the GE 2270 complex.

For instance, it has been observed that a certain type of fish meal (Agras type 99.9 herring fish meal produced by: Esbjerg Fiskeindustri AMBA Fiskerihavnsgade 35, 6700 Esbjerg, Denmark), which at dosages between 1 and 15 g/liter promotes both the increase of total GE 2270 complex productivity and the increase of selective production of factor A, at concentration of 20 g/liter practically inhibits the growth of the microorganism and the production of the antibiotic. Such effect was not observed with the addition of de-fatted fish meal Agras type 99.9.

The flask or vessels containing the standard fermentation medium and the appropriate amount of vitamin B₁₂, after inoculation with the above mentioned culture, are kept at a temperature between 24°C and 37°C, preferably, between 28°C and 30°C, with shaking or stirring for a period sufficiently long for achieving the maximum yield of antibiotic GE 2270 complex. This period is essentially determined by monitoring the fermentation course by analytical procedures, including bioassays, such as paper disc or agar diffusion assays on sensible microorganisms (e.g. Bacillus subtilis or S. aureus), TLC or HPLC procedures commonly utilized in the art (see EP-A 359062).

In general a fermentation period sufficient for achieving the maximum yield of antibiotic GE 2270 under the conditions utilized according to this invention ranges from 3 to 7 days.

The composition of the standard pre-culture and the fermentation media utilized for carrying out the method of this invention contain the nutrient materials usually employed in the art. Certain nutrient materials are however preferred.

Preferred carbon sources are glucose, mannose, galactose, starch, corn meal and the like. Preferred nitrogen sources are ammonia, nitrates, soybean meal, peptone, hydrolyzed casein, meat extract, yeast extract, tryptone, aminoacids, and the like. Among the inorganic salts which can be incorporated in the culture media there are the customary soluble salts capable of yielding sodium, potassium, iron, zinc, cobalt, magnesium, calcium, ammonium, chloride, carbonate, sulfate, phosphate, nitrate and the like ions.

For example, a standard fermentation medium (without considering the addition of vitamin B₁₂ or its analogs) may contain soluble starch, hydrolyzed casein, yeast extract, meat extract, soybean meal, glucose and calcium carbonate. To the nutrient components described above, other materials can be added in case the vitamin B₁₂ is supplemented as a component of a complex material. Fish meal, animal organ extracts, raw extracts from vitamin B₁₂ microbial productions are representative examples of said complex material containing vitamin B₁₂.

A particular effect on both the selectivity in factor A production and the total potency of the fermentation broth has been observed when ammonium sulfate is added to the fermentation medium. The referred concentrations of ammonium sulfate range between 0.5 and 1.5 g/l.

When the fermentation is stopped, the antibiotic GE 2270 complex which is obtained under the general conditions of this invention usually contains more than 80% (HPLC) of factor A. By carrying out the fermentation process under the most preferred conditions final concentrations of total antibiotic GE 2270 complex of the order of 350-500 ppm in the fermentation broth can be obtained, with a ratio of factor A of about 95% (HPLC).

The antibiotic product can be recovered from the fermentation broth and purified according to the methods previously disclosed. (See EP-A 359062). In particular, when the crude GE 2270 contains the factor A in a proportion over 90%, it can be directly purified by crystallization from an organic solvent or a mixture of organic solvents yielding a product which contains less than 5% of the total other factors.

The following examples describes some representative ways to carry out the method of this invention.

### EXAMPLE 1

### Fermentation procedures

1.1) Microorganism: The microorganism used is Planobispora rosea ATCC 53773 or a GE 2270 producing mutant or variant thereof which is stored in frozen culture broths maintained at -80°C for use as working stocks.

The storage medium has the following composition:

| | |
|---|---|
| Soluble starch | 20 g/liter |
| Polypeptone | 5 g/liter |
| Yeast extract | 3 g/liter |
| Meat extract | 2 g/liter |
| Soybean meal | 2 g/liter |
| CaCO₃ | 1 g/liter |

1.2) Fermentation conditions: Pre-cultures are prepared in 500 ml Erlenmeyer flasks by inoculating with 5 ml of the above frozen culture the flasks containing the same medium as above.
The inoculated flasks are incubated for 72 hours at 28°C with shaking (200 rpm), then 4 ml aliquotes of the culture broth are transferred to the flasks containing the following medium supplemented with the selected amount of vitamin B₁₂ or its analogs.

| | |
|---|---|
| Soluble starch | 35 g/liter |
| Hydrolysed casein | 5 g/liter |
| Yeast extract | 8 g/liter |
| Meat extract | 3.5 g/liter |
| Soybean meal | 3.5 g/liter |
| Glucose | 10 g/liter |
| CaCO₃ | 2 g/liter |

This medium is prepared in distilled water and the pH is corrected to 7.2 before sterilization (122°C for 30 minutes). Each medium contains 0.03% of Hodag AFM-5 as antifoaming agent.
The fermentation is carried out for 7 days at 28°C - 30°C with shaking (200 rpm), during which period the fermented broths are monitored for the antibiotic production.

1.3) Vitamin B₁₂ additions:

1.3.1) Vitamin B₁₂ or its analogs are dissolved in distilled water at a starting concentration of 25 mg/ml and are diluted in water. The solutions are sterilized by filtration and added to the fermentation medium described above at the selected concentration.

1.3.2) Fish meals (Agras type 99.9 and Agras type 99.9 de-fatted) are added to the fermentation flasks containing the above medium in the selected amounts.

1.4) HPLC analysis of the antibiotic production:

One volume of the fermented broth is extracted with two volumes of acetonitrile by stirring at room temperature for about 20 minutes and the suspension is centrifuged for 5 minutes at 3.000 rpm.
The solution obtained is used for the HPLC analysis by assuming a dilution ratio 1:3.

### HPLC conditions:

| | |
|---|---|
| Instrument | HP 1082 with UV 254 detector |
| Column | Brownlee RP-18 5µ 22 cm |
| Precolumn | Brownlee RP-18 5µ 1.5 cm |
| Eluent phase A | NaH₂PO₄ 20 mM - CH₃CN 90:10 |
| Eluent phase B | NaH₂PO4 20 mM - CH₃CN 30:70 |
| Gradient type | linear |
| Gradient time | 20 min. from 45% B to 75% B |
| Flow rate | 1.5 ml/min. |
| Injection | 30 µl |

Under the above conditions, all peaks of the HPLC chromatographic profile falling within the retention times (Rₜ) interval from 6.20 to 13.67 minutes are related to the factors of the GE 2270 complex. Factor A shows a Rₜ value of 11.65 minutes.

### EXAMPLE 2

### Effects of addition of vitamin B₁₂ or its analogs

2.1) The following Table 1 reports two experiments showing the effects of the addition of vitamin B₁₂ at various concentrations to the fermentation medium. Estimations are made by HPLC methods.

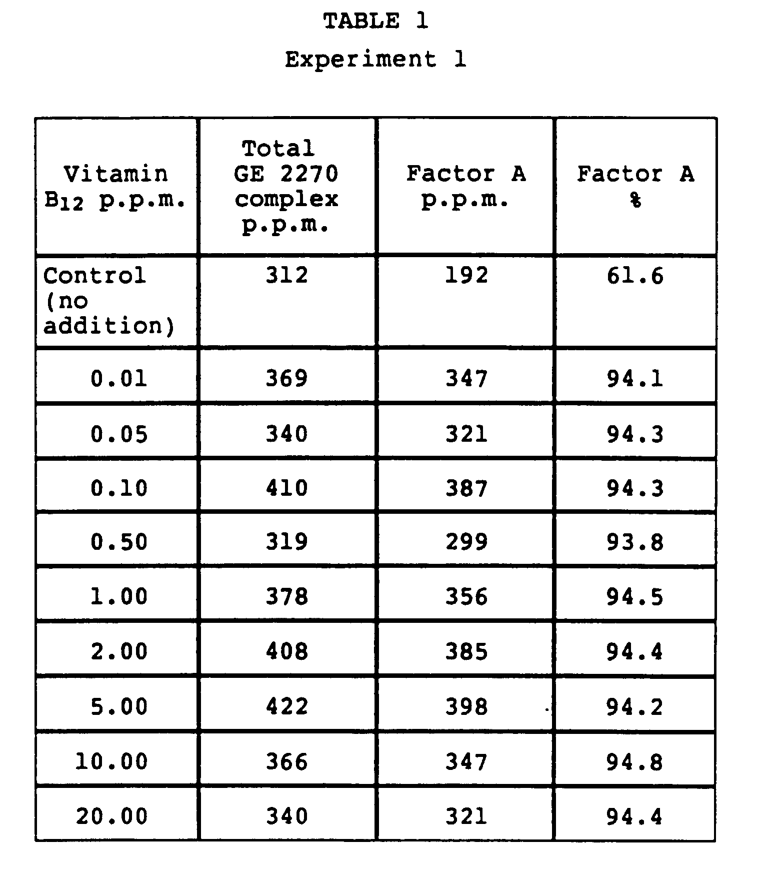

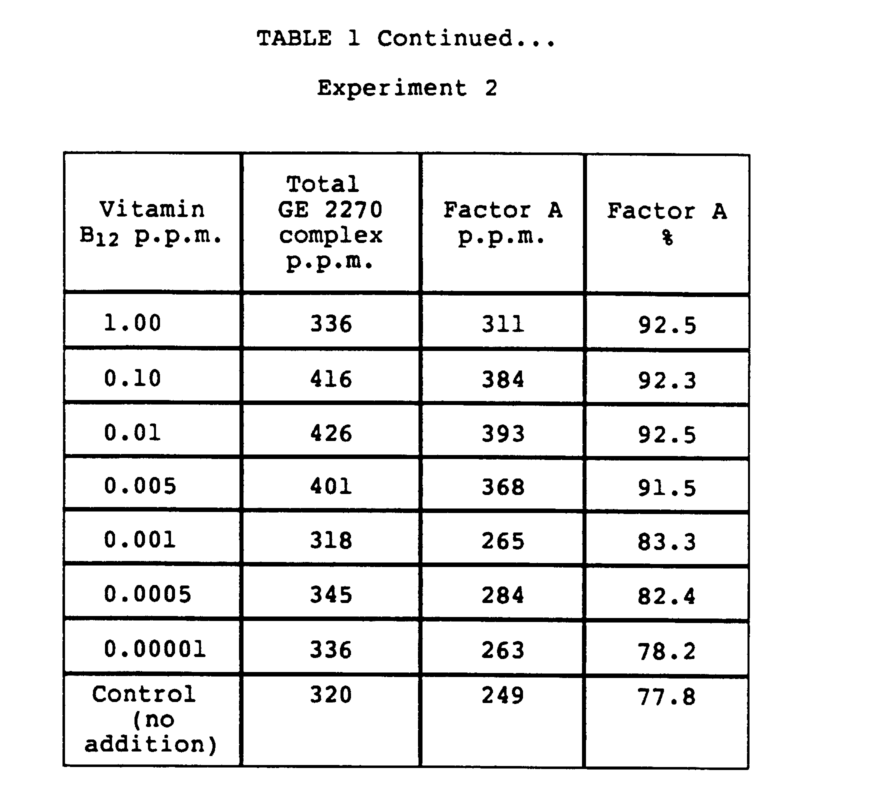

2.2) The following Table 2 shows the effect of the addition of methylcobalamin at various concentrations to the fermentation medium.

**TABLE 2**

| Methyl-cobalamin | Factor A p.p.m. | Factor A % |
|---|---|---|
| Control (no addition) | 87 | 47.0 |
| 0.001 | 235 | 83.1 |
| 0.01 | 280 | 91.6 |
| 0.10 | 297 | 93.2 |
| 1.00 | 290 | 92.2 |

2.3) The following Table 3 shows the effects of the addition of various amounts of fish meal to the fermentation medium.

**TABLE 3**

| Fish meal | Factor A p.p.m. | Factor A % |
|---|---|---|
| Control (no addition) | 99 | 46.5 |
| Agras type 99.9 (g/l) | | |
| 1 | 206 | 68.8 |
| 2.5 | 282 | 82.2 |
| 5 | 310 | 88.8 |
| 7.5 | 340 | 88.4 |
| 10 | 373 | 89.6 |
| 15 | 300 | 91.6 |
| 20 | 4 | - |
| 30 | 1 | - |
| Agras type 99.9 de-fatted (g/l) | | |
| 5 | 355 | 93.9 |
| 10 | 466 | 93.9 |
| 20 | 445 | 92.8 |

## Claims

1. A fermentation process for the production of antibiotic GE 2270 complex in high yield and selectively enriched in the predominant factor A by cultivation of the strain Planobispora rosea ATCC 53773 or any antibiotic GE 2270 producing mutant or variant thereof under submerged aerobic conditions in the presence of assimilable sources of carbon, nitrogen and inorganic salts characterized in that vitamin B₁₂, or its analogs having vitamin B₁₂ - like activity wherein the cyano group is replaced by another ligand, is added to the fermentation medium.

2. A process as in claim 1 wherein the amount of vitamin B₁₂ or its analogs added to the fermentation medium ranges between 0.0005 and 5 ppm, preferably, between 0.005 and 1 ppm.

3. A process as in any of claim 1 and 2 wherein the vitamin B₁₂ or its analogs are selected from vitamin B₁₂, hydroxocobalamin, 5'-deoxyadenosyl-cobalamin and methylcobalamin.

4. A process as in any of claims 1 and 2 wherein vitamin B₁₂ is added as a component of a more complex material acceptable for the fermentation conditions required for the production of the antibiotic GE 2270.

5. A process as in claim 4 wherein the complex material is fish meal or de-fatted fish meal.

6. A process as in claim 5 wherein the amount of fish meal or de-fatted fish meal added to the fermentation medium ranges from 1 to 15 g/l.

7. A process as in claim 6 wherein the fish meal is Agras type 99.9 or de-fatted Agras type 99.9.

8. A process as in any of claims 1 to 7 wherein ammonium sulfate is added to the fermentation medium, preferably, at a concentration ranging between 0.5 and 1.5 g/l.

## Patentansprüche

1. Fermentationsverfahren zur Produktion eines Antibiotikum GE 2270-Komplexes mit hoher Ausbeute und selektiver Anreicherung des vorherrschenden Faktors A durch Züchtung des Stammes Planobispora rosea ATCC 53773 oder einer beliebigen, das Antibiotikum GE 2270 produzierenden Mutante oder einer Variante davon unter aeroben Submersbedingungen in Gegenwart von assimilierbaren Kohlenstoff-, Stickstoff- und anorganischen Salz-Quellen, dadurch gekennzeichnet, daß Vitamin B₁₂ oder seine Analogen mit Vitamin B₁₂-ähnlicher Aktivität, bei denen die Cyanogruppe durch einen anderen Liganden ersetzt ist, zu dem Fermentationsmedium hinzugefügt werden.

2. Verfahren nach Anspruch 1, wobei die Menge an zu dem Fermentationsmedium hinzugefügten Vitamin B₁₂ oder seinen Analogen im Bereich von 0,0005 bis 5 ppm liegt, vorzugsweise zwischen 0,005 und 1 ppm.

3. Verfahren nach Anspruch 1 oder 2, wobei das Vitamin B₁₂ oder seine Analoge ausgewählt sind aus Vitamin B₁₂, Hydroxycobalamin, 5'-Desoxyadenosyl-cobalamin und Methylcobalamin.

4. Verfahren nach Anspruch 1 oder 2, wobei Vitamin B₁₂ als Bestandteil eines komplexeren Materials hinzugefügt wird, das für die zur Produktion des Antibiotikums GE 2270 erforderlichen Fermentationsbedingungen geeignet ist.

5. Verfahren nach Anspruch 4, wobei das komplexe Material Fischmehl oder entfettetes Fischmehl ist.

6. Verfahren nach Anspruch 5, wobei die zu dem Fermentationsmedium hinzugefügte Menge an Fischmehl oder entfettetem Fischmehl im Bereich von 1 bis 15 g/l liegt.

7. Verfahren nach Anspruch 6, wobei das Fischmehl vom Agras-Typ 99.9 oder vom Agras-Typ 99.9, entfettet, ist.

8. Verfahren nach einem der Ansprüche 1 bis 7, wobei Ammoniumsulfat zu dem Fermentationsmedium hinzugefügt wird, vorzugsweise bei einer Konzentration im Bereich von 0.5 bis 1.5 g/l.

## Revendications

1. Procédé de fermentation destiné à la production, avec un rendement élevé, du complexe antibiotique GE 2270 sélectivement enrichi en facteur A prédominant comprenant la culture de la souche Planobispora rosea déposée auprès de l'ATCC sous le numéro 53773 ou toute forme mutante ou variante de celle-ci produisant l'antibiotique GE 2270 dans des conditions d'immersion en aérobiose en présence de sources assimilables de carbone, d'azote et de sels inorganiques, caractérisé en ce que la vitamine B₁₂ ou ses analogues possédant une activité de type vitamine B₁₂ dans lesquels le groupe cyano est remplacé par un autre ligand, est ajouté au milieu de fermentation.

2. Procédé selon la revendication 1 dans lequel la quantité de vitamine B₁₂ ou de ses analogues ajoutée au milieu de fermentation se situe dans une plage de 0,0005 à 5 ppm, de préférence de 0,005 à 1 ppm.

3. Procédé selon l'une des revendications 1 ou 2 dans lequel la vitamine B₁₂ ou ses analogues sont choisis parmi la vitamine B_{12,} l'hydroxocobalamine, la 5'-désoxyadénosylcobalamine et la méthylcobalamine.

4. Procédé selon l'une des revendications 1 ou 2 dans lequel la vitamine B12 est ajoutée sous la forme d'un composant d'un matériau plus complexe acceptable dans les conditions de fermentation requises pour la production de l'antibiotique GE 2270.

5. Procédé selon la revendication 4 dans lequel le matériau complexe est une farine de poisson ou une farine de poisson dégraissée.

6. Procédé selon la revendication 5 dans lequel la quantité de farine de poisson ou de farine de poisson dégraissée ajoutée au milieu de fermentation est comprise entre 1 et 15 g/l.

7. Procédé selon la revendication 6 dans lequel la farine de poisson est une farine Agras type 99,9 ou Agras type 99,9 dégraissée.

8. Procédé selon l'une des revendications 1 à 7 dans lequel du sulfate d'ammonium est ajouté au milieu de fermentation, de préférence à une concentration comprise entre 0,5 et 1,5 g/l.
